(19) Europäisches Patentamt / European Patent Office / Office européen des brevets

(11) **EP 2 490 180 B1**

(12) **EUROPEAN PATENT SPECIFICATION**

(45) Date of publication and mention
of the grant of the patent:
**24.07.2013   Bulletin 2013/30**

(51) Int Cl.:
***G06T 11/00*** *(2006.01)*

(21) Application number: **12155921.5**

(22) Date of filing: **17.02.2012**

(54) **Medical image processing apparatus and medical image imaging method**

Vorrichtung und Verfahren zur medizinischen Bildgebung

Dispositif et procédé pour l'imagerie medicale

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.02.2011   US 201113029318**

(43) Date of publication of application:
**22.08.2012   Bulletin 2012/34**

(73) Proprietors:
• **Kabushiki Kaisha Toshiba**
**Tokyo 105-8001 (JP)**
• **Toshiba Medical Systems Corporation**
**Otawara-shi**
**Toshigi**
**324-8550 (JP)**

(72) Inventors:
• **Zou, Yu**
**Naperville, Illinois 60563 (US)**
• **Hein, Ilmar**
**Chicago, Illinois 60601 (US)**

(74) Representative: **Granleese, Rhian Jane**
**Marks & Clerk LLP**
**90 Long Acre**
**London WC2E 9RA (GB)**

(56) References cited:
**US-A- 5 124 914**

• **HENGYONG YU ET AL: "Data Consistency Based Rigid Motion Artifact Reduction in Fan-Beam CT", IEEE TRANSACTIONS ON MEDICAL IMAGING, IEEE SERVICE CENTER, PISCATAWAY, NJ, US, vol. 26, no. 2, 1 February 2007 (2007-02-01), pages 249-260, XP011161756, ISSN: 0278-0062**
• **KACHELRIESS MARC ET AL: "Kymogram detection and kymogram-correlated image reconstruction from subsecond spiral computed tomography scans of the heart", MEDICAL PHYSICS, AIP, MELVILLE, NY, US, vol. 29, no. 7, 1 July 2002 (2002-07-01), pages 1489-1503, XP012011846, ISSN: 0094-2405, DOI: 10.1118/1.1487861**

## Description

FIELD

Embodiments described herein relate generally to a medical imaging apparatus and a medical imaging method.

BACKGROUND

In general, medical imaging data generally assumes a stationary object. That is, it is assumed that the data has been acquired while a patient makes no motion. The motion of the object includes both body movements and organ movements during the data acquisition. When the images are reconstructed based upon acquired data reflecting some motion, the reconstructed medical images substantially suffer from motion artifacts so that these images could lead the doctors to an inaccurate diagnosis.

One approach in improving the image quality is to identify certain portions of the acquired data that reflect the least amount of motion. Since the identified portions of the data have the least amount of motion, when the medical images are reconstructed from the acquired data with the least motion, the reconstructed medical images are also least affected by the motion artifacts. This approach utilizes a motion index to identify certain data portions that had been acquired while the least amount of motion took place.

Some existing motion indexes are based upon the Helgason-Ludwig consistency condition, complementary rays and a sinogram. Unfortunately, these techniques are either inapplicable to different situations or inaccurate over various projection data sets. Thus, a new motion index remains to be desired for various applications and projection data sets.

HENGYONG YU ET AL: "Data Consistency Based Rigid Motion Artifact Reduction in Fan-Beam CT", IEEE TRANSACTIONS ON MEDICAL IMAGING, vol. 26, no. 2, 1 February 2007, pages 249-260, discloses a method for recovering motion parameters directly from inconsistencies in tomographic projection data using the *Helgason-Ludwig* consistency condition.

KACHELRIESS MARC ET AL: "Kymogram detection and kymogram-correlated image reconstruction from subsecond spiral computed tomography scans of the heart", MEDICAL PHYSICS, vol. 29, no. 7, 1 July 2002, pages 1489-1503, discloses a method for extracting cardiac motion parameters from measured raw data by tracking the center of mass in a scanned slice.

US 5,124,914 discloses the well-known *Grangeat* formula for cone-beam reconstruction which relates the cone-beam X-ray transform of an object function to the first derivative of its Radon transform.

BRIEF DESCRIPTION OF THE DRAWINGS

FIG. 1 is a diagram illustrating an X-ray CT imaging system according to an embodiment;
FIG. 2 is a diagram illustrating a method of determining a motion index according to this embodiment;
FIG. 3 is a diagram illustrating three points on a predetermined X-ray source trajectory in determining a motion map according to this embodiment;
FIG. 4 is another diagram illustrating a method of determining a motion index according to this embodiment;
FIG. 5 is still another diagram illustrating a method of determining a motion index according to this embodiment;
FIG. 6 illustrates a numerical simulation to show the accuracy and temporal resolution of the motion index according to this embodiment;
FIG. 7A is a graph showing changes in derivative of planar integral (DPI) at the first rotation during four consecutive rotations over a circular trajectory in a clinical cardiac scan according to this embodiment;
FIG. 7B is a graph showing changes in derivative of planar integral (DPI) at the second rotation during four consecutive rotations over a circular trajectory in a clinical cardiac scan according to this embodiment;
FIG. 7C is a graph showing changes in derivative of planar integral (DPI) at the third rotation during four consecutive rotations over a circular trajectory in a clinical cardiac scan according to this embodiment;
FIG. 7D is a graph showing changes in derivative of planar integral (DPI) at the fourth rotation during four consecutive rotations over a circular trajectory in a clinical cardiac scan according to this embodiment;
FIG. 8 illustrates changes in DPI over the course of one heart beat according to this embodiment;
FIG. 9 illustrates the reconstructed images according to the selected half scan projection data of FIG. 8;
FIG. 10 is a diagram illustrating arrangement of a positron emission tomography (PET) imaging system 100 according to this embodiment;
FIG. 11 is a diagram illustrating a detector ring included in the PET imaging system in FIG. 10;

FIG. 12 is a diagram illustrating a single photon emission computed tomography (SPECT) imaging system according to this embodiment;

FIG. 13 is a diagram illustrating a gamma camera which is included in the SPECT imaging system in FIG. 12 and directly measures gamma rays emerging from a region of interest in an object S; and

FIG. 14 is a flowchart illustrating a typical procedure for processing according to this embodiment.

DETAILED DESCRIPTION

One approach in improving the image reconstruction quality is to identify portions of the data involving the least amount of motion. That is, one approach is to identify certain portions of projection data that had been acquired while the least amount of motion took place in the region of interest. Since the identified portions of projection data have the least amount of motion, the images reconstructed from the identified projection data are likely to be least affected by the motion artifacts. In other words, the approach of this embodiment utilizes the consistent portion of the projection data. More specifically, a similar reconstruction approach is to utilize the projection data that are centered on the above described consistent range of data. The above identified projection data substantially reduces motion artifacts in the reconstructed images.

Note that projection data are managed for each view. A set of projection data for each view will be referred to as a projection data set. A portion or portions of projection data indicate a projection data set, of all the projection data acquired during an imaging period, which is associated with a predetermined view. In this embodiment, "consistent" means the anatomical identity between substances on X-ray transmission paths in different views. In other words, "consistent" means that variations in motion index value due to the motion of the object are relatively small throughout a plurality of views to be compared. Consistent projection data does not include many motion artifact components of the object throughout a plurality of views to be compared. Motion indices according to the embodiment will be described in detail later. A consistent portion of projection data indicates a projection data set, of all the projection data, which belongs to a view range with relatively small variations in the amount of motion. On the other hand, "inconsistent" in the embodiment indicates that variations in motion index value due to the motion of the object are relatively large throughout a plurality of views to be compared. An inconsistent portion of projection data indicates a projection data set, of all the projection data, which belongs to a view range with relatively large variations in the amount of motion.

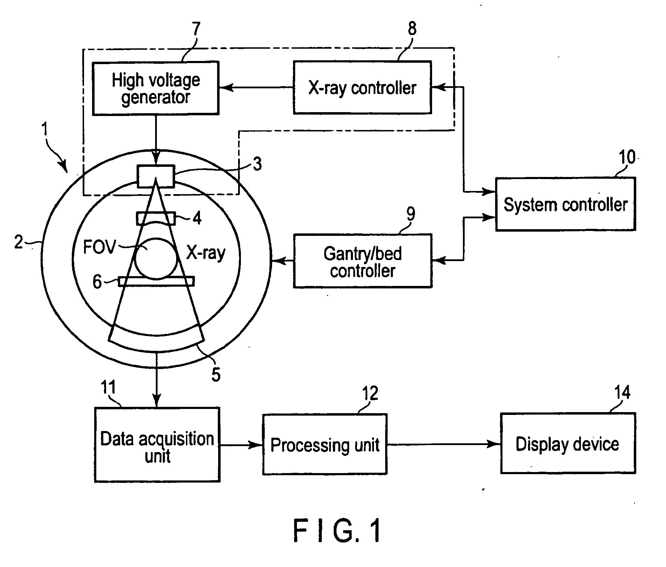

FIG. 1 is a diagram illustrating the arrangement of an X-ray CT imaging system according to this embodiment. The X-ray CT imaging system according to the embodiment includes a gantry 1, which accommodates an X-ray source 3 and a two-dimensional array type X-ray detector 5. The X-ray source 3 and the X-ray detector 5 are installed on a rotating ring 2 at opposite sides of an object. The object is laid on a sliding bed 6. The rotating ring 2 rotates around a predetermined rotational axis while the sliding bed 6 moves along the rotational axis at a predetermined speed. A gantry/bed controller 9 synchronously controls the rotation of the rotating ring 2 and the sliding movement of the sliding bed 6. Projection data is acquired while the X-ray source 3 travels either a helical path in a helical scanning mode or a circular path in a circular scanning mode.

The X-ray source 3 emits a cone-beam or approximately cone-shaped X-ray flux that is directed onto the object through an X-ray filter 4. An X-ray controller 8 supplies a trigger signal to a high voltage generator 7. The high voltage generator 7 applies high voltage to the X-ray source 3 upon receiving the trigger signal. A system controller 10 exerts an overall control over both the X-ray controller 8 and the gantry/bed controller 9 so that X-rays are emitted continuously or intermittently at fixed angular intervals from the X-ray source 3 while the rotating ring 2 and the sliding bed 6 are respectively in predetermined rotation and motion.

X-rays that have passed through the object are detected as electrical signals by the X-ray detector 5. In the X-ray detector 5, a plurality of detector elements are arranged in one dimensional row. The plurality of rows are stacked to construct a two-dimensional array. For example, each detector element corresponds to one channel. A data acquisition unit 11 amplifies the output signal from the X-ray detector 5 for each channel and converts to a digital signal (projection data).

A processing unit 12 subsequently performs various processing tasks on the projection data that has been transferred from the data acquisition unit 11. For example, the processing unit 12 performs on the projection data desired operation including data sampling, data shifting, filtering, selection, backprojection and reconstruction.

The processing unit 12 calculates backprojection data based upon the projection data. In a circular scanning system using a cone-beam of X-rays, the imaging region (effective field of view (FOV)) is of a cylindrical shape of radius R centered on the axis of rotation. The processing unit 12 determines a plurality of voxels (three-dimensional pixels) in this imaging region and calculates the backprojection data for each voxel. The processing unit 12 generates three-dimensional image data or tomographic image data based upon the backprojection data. A display device 14 displays a three-dimensional image or tomographic image according to the three-dimensional image data.

As will be later described in details, the processing unit 12 also selects a certain portion of the projection data before backprojection and reconstruction. For example, the processing unit 12 selects before backprojection and reconstruction a portion or portions of the projection data experiencing the least amount of motion according to a predetermined motion index. The projection data selection is an important task for improving the image quality of non-stationary organs such as the heart and lungs.

As well known, an object or patient attenuates the X-ray beam (ray). The attenuated X-ray beam intensity I at the detector element k is defined in Equation (1) as follows:

$$I_k = I_k^0 \exp\left(- \int_\ell \mu(x) dx\right) \qquad (1)$$

where $\mu(x)$ is the attenuation function to be reconstructed. $I_k^0$ is the X-ray beam intensity before attenuation by $\mu(x)$ after penetrating through the X-ray filter. $\int_\ell \mu(x) dx$ is the line integral of $\mu(x)$ along the line t. Mathematically, $\mu(x)$ can be reconstructed as a set of line integrals respectively corresponding to a plurality of the lines $\ell$. Therefore, measured X-ray beam intensities $I_k^0$ are to be converted into line integrals as follows in Equation (2):

$$\int_\ell \mu(x) dx = \ln\left(I_k^0\right) - \ln\left(I_k\right) \qquad (2)$$

X-ray tomographic imaging includes the following three main steps, data acquisition, data processing and data reconstruction. In data acquisition, the X-ray beam intensity data are acquired at each detector element of the X-ray detector 5 at each predefined angular view position $\beta$ while the gantry 1 is rotated. The X-ray detector 5 measures incident X-ray by integrating energy (charge) or counting X-ray photons. The detected incident X-rays are converted into an electric signal. Then, the electric signal is transferred from a rotating part of the gantry 1 to a stationary part though the rotating ring.

The projection data from the data processing unit 11 are converted, by data processing by the processing unit 12, from X-ray intensity measurements to the projection data corresponding to line integrals according to Equation (2). Also, various corrections steps are applied to reduce undesirable effects of physical phenomena such as scattering and X-ray beam hardening to compensate the non-uniform response function of each detector element, and also to reduce noise.

In this embodiment, the processing unit 12 performs data selection processing as illustrated in the following embodiments. Data selection is related to motion alone. Motion is determined by certain predetermined indexes such as the derivative of plane integrals (DPI). Note that the data selection processing to be performed is not limited to this, and may be implemented in a variety of different ways.

In clinical applications, two major types of motion include motion of the heart (cardiac motion) and lung motion. Cardiac data is typically obtained over several heart beats at a slow helical pitch so that redundant data are sufficiently available for gated reconstruction. During the data acquisition, an electrocardiogram (ECG) is also obtained for information on cardiac motion.

Note that cardiac data means projection data acquired by the data acquisition unit 11 in imaging of the heart. Gated reconstruction in cardiac imaging is typically ECG gated reconstruction.

Still in relation to selecting a certain portion of the projection data based upon motion, lung data is acquired in the following manner. Lung data is typically acquired at a faster helical pitch so that some redundant data are available, but they are not usually enough for gated reconstruction. Lung data may be sufficient only for a very limited gated reconstruction. Unlike ECG for the cardiac motion, no external motion information is generally available for reconstruction with respect to lung data. In addition to the above described type of the clinical data, motion may be caused by the movement of a patient during the data acquisition. This body motion of the patient may be combined with the organic movement.

Note that lung data means projection data acquired by the data acquisition unit 11 in imaging of the lung. Gated reconstruction in pulmonary imaging is typically respiratory gated reconstruction.

In both cardiac and lung cases, available redundant data is selected based on object motion. The main idea is to use a predetermined motion index such as DPI so that for example, the consistent portions of the projected data are selected. In the following disclosure on the exemplary embodiments, the processing unit 12 or its equivalent device performs tasks related to the calculation of motion index values based on the projection data and the above described selection of the consistent portions of the projection data based upon the motion index values. Similarly, in the following example, a processing step is used to typically include steps or sub steps that are related to the above described determination and selection functions.

To understand the motion index as used in the embodiments, the following relationship is explained among line integrals, plane integrals and the DPI. A 3D cone-beam projection of an image function f(·) can be written as Equation (3) below:

$$g\left(\bar{a}(\lambda),\hat{\beta}\right) = \int_0^\infty f\left(\bar{a}(\lambda) + r\hat{\beta}\right)dr \qquad (3)$$

Assume that A⁻ and A^ will respectively represent $\overline{A}$ and $\hat{A}$ hereinafter except for those in the equations and the accompanying drawings.

In this case, g(a⁻(λ),β^) is projection (line integral), a⁻(λ) represents the X-ray source position, β ^ = e⁻ (α,γ) indicates a unit vector in 3D image space determined by the X-ray source position a⁻(λ), cone angle α, and fan angle γ or β^, and r is a distance parameter from an X-ray source to a point in the object.

Planar integral is defined in Equation (4) below:

$$p(1,\hat{n}) = \int_{R3} f(\bar{x})\delta(1 - \bar{x}\cdot\hat{n})d\bar{x} \qquad (4)$$

where n^ is the normal of the plane and 1 is the distance from the origin to the plane.

To derive Grangeat's formula, consider the following:

$$\int_{S2} g\left(\bar{a}(\lambda),\hat{\beta}\right)\delta'\left(\hat{\beta}\cdot\hat{n}\right)d\hat{\beta} = \int_{S2} d\hat{\beta} \int_0^\infty f\left(\bar{a}(\lambda) + r\hat{\beta}\right)dr\delta'\left(\hat{\beta}\cdot\hat{n}\right) \qquad (5)$$

To calculate the right-hand side of Equation (5), g(a⁻(λ),β^) of Equation (5) is substituted by the right-hand side of Equation (3). Furthermore, x⁻ = a⁻ + rβ^ is assumed to have Equation (6) below.

$$= \int_{S2} d\hat{\beta} \int_0^\infty r^2 dr f(\bar{x})\frac{1}{r^2}\delta'\left(\frac{1}{r}(\bar{x} - \bar{a}(\lambda))\cdot\hat{n}\right) \qquad (6)$$

From Equation (4), by eliminating r, Equation (7) is obtained as below:

$$= \int_{R3} d\bar{x} f(\bar{x})\delta'\left((\bar{x} - \bar{a}(\lambda))\cdot\hat{n}\right) \qquad (7)$$

On the other hand, by differentiating Equation (4), Equation (8) is calculated below as the DPI:

$$\frac{\partial p(1,\hat{n})}{\partial 1} = \int_{R3} f(\bar{x})\delta'(1 - \bar{x}\cdot\hat{n})d\bar{x} = -\int_{R3} f(\bar{x})\delta'(\bar{x}\cdot\hat{n} - 1)d\bar{x} \qquad (8)$$

By comparing Equations (7) and (8) and assuming that the condition a⁻(λ)·n^=1 is true, Grangeat's formula in Equation (9) is derived as below. According to Grangeat's formula, DPI is related to the cone-beam projection data as expressed in Equation (5).

$$\frac{\partial p(1,\hat{n})}{\partial 1}\bigg|_{1=\bar{a}(\lambda)\cdot\hat{n}} = -\int_{S2} g\left(\bar{a}(\lambda),\hat{\beta}\right)\delta'\left(\hat{\beta}\cdot\hat{n}\right)d\hat{\beta} \qquad (9)$$

where, g(a⁻(λ),β^) is projection (line integral). a⁻(λ) represents the X-ray source position. β ^=e⁻(α,γ) indicates a unit vector in 3D image space defined by the X-ray source position a⁻(λ) and cone angle α and fan angle γ or β^.

Thus, Equation (9) can also be written as Equation (10) below:

$$\left.\frac{\partial p(1,\hat{n})}{\partial 1}\right|_{1=\bar{a}(\lambda)\cdot\hat{n}} = -\int_{S^2} g(\lambda,\alpha,\gamma)\delta'(\hat{n}\cdot\hat{e}(\alpha,\gamma))d\hat{e}(\alpha,\gamma) \qquad (10)$$

As expressed in Equation (10), the DPI is calculated from projection data in various views. The calculated DPI values are the same if the data are consistent. This is because the planar integral is dependent only on image function f(x⁻). DPI has the same feature. In CT imaging, it is assumed that the object is stationary and the image function is the same during a scan. Therefore, DPI is uniform on a plane for a specific image function (object). It is not dependent on how and where the DPI is obtained (i.e., through line integrals at different locations). The difference in DPI on the same plane from different X-ray source positions necessarily indicates a change in the image function during the scan (i.e., moving of the X-ray source position). In other words, the DPI values are the same if there is no motion.

That is, if projection data are consistent throughout a plurality of views to be compared, a plurality of DPIs associated with the plurality of views are theoretically identical. In other words, if the attenuation sum on the transmission path of cone-beam X-rays on a test plane remains the same in a plurality of views, the projection data are consistent throughout the plurality of views.

As is obvious from the above discussion, DPIs vary depending on motion components perpendicular to a test plane. If, for example, the patient makes motion in a direction crossing a test plane throughout two views, the difference between the two DPIs associated with the two views is ideally non-zero. In contrast, if the patient makes motion only in a direction parallel to a test plane throughout two views, the difference between the two DPIs associated with the two views is ideally almost zero. That is, DPIs associated with a given test plane have direction dependence in accordance with the orientation of the test plane in association with the detection of body motion.

The embodiments use the above feature of DPI as a motion index. The DPI values in two neighboring views are used as a motion index and compared to examine the consistency. The temporal resolution is determined by the acquisition time of two views. However, due to aliasing and noise, the comparison may not be reliable for the two immediately neighboring views. For this reason, for example, a running average of DPIs over a predetermined number of views such as 63 consecutive views may be used to evaluate an amount of motion in order to reduce undesirable effects of aliasing error and noise.

The temporal resolution substantially improves in detecting a sudden or quick movement when DPIs are used as a motion index. Other motion indexes such as complementary views require that the two corresponding projection data sets are acquired about 180 degrees apart. During the X-ray source travelling over the 180 degrees, the object such as the heart may experience certain small reciprocating movements. In other words, by the time when the X-ray source completes its travels over the 180 degrees, a certain part of an object moves to another position and comes back to the original position. Thus, a time lag in acquiring the projection data causes a failure in capturing certain fast motion in the object.

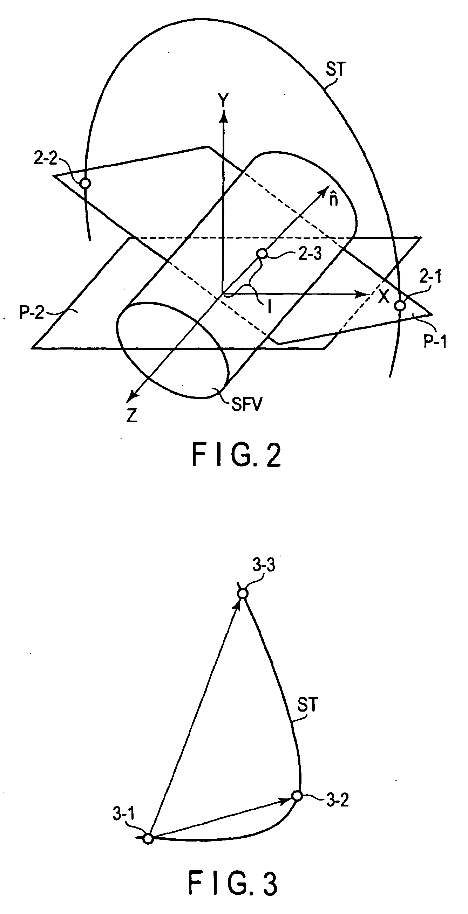

FIG. 2 is a diagram illustrating a method of determining a motion index according to the embodiment. The motion index is used for selecting consistent portions of projection data. Cone-beam X-rays are typically emitted from an X-ray source 3 that travels along a predetermined X-ray source trajectory ST. At each projection angle β with respect to the X-axis, the X-rays are emitted with a cone angle α and a fan angle γ and pass through an object with a scanned field of view (SFV). Thus, the projection data are acquired by the data acquisition unit 11 in the above described manner.

As shown in FIG. 2, a plane P-1 is a test plane and includes a point 2-3. The test plane P-1 is perpendicular to a unit vector n^. The unit vector n^ intersects the test plane P-1 at a distance 1 from the origin of the X-, Y- and Z-axes. A plane P-2 is a plane defined by the X- and Z-axes. Assuming that two points 2-1 and 2-2 on a 3D X-ray source trajectory ST are located on the test plane P-1. The projection data set regarding to the view at the point 2-1 and the projection data set regarding to the view at the point 2-2 are acquired. For the test plane P-1, the DPI are calculated from the cone-beam projection data at the two points 2-1 and 2-2. The DPI from the point 2-1 is equal to the DPI from the point 2-2 if the projection data at the two points 2-1 and 2-2 are consistent (i.e. no motion). The difference in the two DPI values represents the inconsistency of the two projection data sets from the two views. Assuming that the inconsistency is primarily caused by motion, the difference in the two DPI values defines an amount of motion and is used as a motion index.

In other words, the data acquisition unit 11 acquires projection data sets at the points 2-1 and 2-1 during an imaging period. The processing unit 12 sets the test plane P-1 including the points 2-1 and 2-2, calculates the DPI on the test plane P-1 at the point 2-1 by using the projection data set at the point 2-1, and calculates the DPI on the test plane P-1 at the point 2-2 by using the projection data set at the point 2-2. DPI is calculated from, for example, a projection data set according to Equation (10). More specifically, first of all, the processing unit 12 calculates a plane integral on the test plane P-1 at the point 2-1 based on the projection data set at the point 2-1, and calculates a plane integral on the test plane P-1 at the point 2-2 based on the projection data set at the point 2-2. In this case, an integral plane regarding to the plane integral at the point 2-1 and an integral plane regarding to the plane integral at the point 2-2 are the same plane, i.e., the test plane P-1. A plane integral is calculated by, for example, performing linear integration of line integral values from a plurality of detection elements included in a detection element array on the X-ray detector 5 crossing the test plane P-1 along the detection element array. The processing unit 12 then calculates the DPI at the point 2-1 by using the plane integral at the point 2-1, and calculates the DPI at the point 2-2 by using the plane integral at the point 2-2. The processing unit 12 calculates the difference between the DPI at the point 2-1 and the DPI at the point 2-2. The calculated difference is used as a motion index for the evaluation of the amount of motion of the object.

Note that the two points 2-1 and 2-2 shown in FIG. 2 are 180 degrees apart. Since the point 2-1 must travel along the X-ray source trajectory ST to reach the point 2-2 over a half rotation of the gantry, the associated projection data at the two points 2-1 and 2-2 have a corresponding amount of relatively long time difference as compared with a case in which the angular interval between the point 2-1 and the point 2-2 is less than 180 degrees. In other words, the projection data sets at the two points 2-1 and 2-2 do not have an appropriate temporal resolution.

The above has described the method of using DPIs for the evaluation of motion between two points. However, the motion evaluation method according to this embodiment is not limited to only two points and can be applied to any number of points equal to or more than three. As an example of the method of evaluating body motion between three or more points, a method of using DPIs for the evaluation of body motion between three points will be described. In this case, it is preferable to use, for example, a motion map for the evaluation of body motion between three points.

FIG. 3 is a diagram illustrating three points on an X-ray source trajectory in calculating a motion map according to this embodiment. Assuming that a 3D X-ray source trajectory ST, and three points 3-1, 3-2 and 3-3 on the 3D X-ray trajectory ST decide a test plane. As already described above with respect to Equations (3) through (11), the DPIs are related to the line integrals according to Grangeat's formula. The DPIs are determined for the three points 3-1, 3-2, and 3-3, respectively. Typically, if the projection data sets at the three points 3-1, 3-2 and 3-3 were consistent, the three DPI values would be identical. On the other hand, if the data were not consistent, the difference in DPI values would measure the data inconsistency or an amount of motion.

In other words, the data acquisition unit 11 acquires projection data sets at three points 3-1, 3-2, and 3-3 during an imaging period. The processing unit 12 sets a test plane including the three points 3-1, 3-2, and 3-3. The processing unit 12 then calculates DPI at the point 3-1 on the test plane by using projection data set at the point 3-1 according to the above method. Likewise, the processing unit 12 calculates DPI at the point 3-2 on the test plane by using projection data set at the point 3-2, and calculates DPI at the point 3-3 on the test plane by using projection data set at the point 3-3. The processing unit 12 then calculates a motion map based on the DPIs at the points 3-1, 3-2, and 3-3.

Assuming that the projection data inconsistency is due to motion, a motion map M according to this embodiment is defined by Equation (11) below:

$$M(\lambda_1, \lambda_3)$$

$$= \frac{1}{\lambda_3 - \lambda_1} \int_{\lambda_1}^{\lambda_3} \left[ \left| \frac{\partial p(l, \hat{n})}{\partial l} \right|_{l=\bar{a}(\lambda_1)} - \frac{\partial p(l, \hat{n})}{\partial l} \Big|_{l=\bar{a}(\lambda_2)} \right| + \left| \frac{\partial p(l, \hat{n})}{\partial l} \right|_{l=\bar{a}(\lambda_3)} - \frac{\partial p(l, \hat{n})}{\partial l} \Big|_{l=\bar{a}(\lambda_2)} \right| d\lambda_2 \quad (11)$$

where $M(\lambda 1, \lambda 3)$ indicates the difference in DPI among the three points 3-1, 3-2 and 3-3. That is, the difference in DPI between the two points 3-1 and 3-3 is determined based upon the sum of DPI difference between the two points 3-1 and 3-2 and DPI difference between the two points 3-2 and 3-3. If the two DPI differences were each zero, the sum of the two DPI difference would also be zero, indicating that the projection data is consistent. On the other hand, if either or both of the two DPI differences were not zero, the sum of the two DPI difference would also be non-zero, indicating that the projection data is inconsistent.

In the above description about the method of using DPIs for the evaluation of motion between two points, the two points are spaced apart from each other by 180 degrees. However, this embodiment is not limited to this. The angular distance between the two points may be less than 180 degrees. A method of evaluating motion between two points spaced apart from each other by less than 180 degrees will be described next.

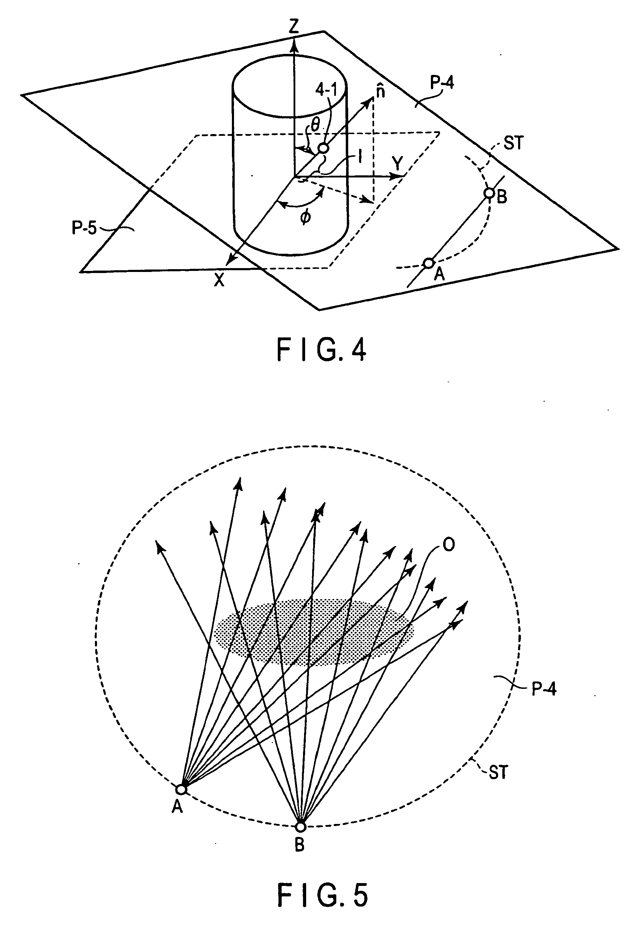

FIG. 4 is another diagram illustrating a method of determining a motion index according to this embodiment. The motion index is used for selecting consistent portions of projection data. Typically, cone-beam X-rays are emitted from the X-ray source 3 that travels along a predetermined X-ray source trajectory ST. At each projection view, the X-rays are emitted with a cone angle $\alpha$ and a fan angle $\gamma$ and pass through an object with a scanned field of view (SFV). Thus, the projection data are acquired by the data acquisition unit 11 in the above described manner.

As shown in FIG. 4, a plane P-4 is a test plane and includes a point 4-1. The test plane P-4 is perpendicular to a unit vector n^. The unit vector n^ intersects the test plane P-4 at angles $\phi$ and $\theta$ with a distance 1 from the origin of the axes X, Y and Z. A plane P-5 is defined by the X and Z axes. Assuming that two points A and B on a 3D X-ray source trajectory ST are located on the test plane P-4. That is, any two points define a family of planes that are also referred to as test planes. Note that a set of two points is a combination of arbitrary points A and B on the 3D X-ray source trajectory ST. Although a single test plane P-4 is only illustrated in FIG. 4, there is in fact a family of test planes. In other words, for the respective test planes, DPIs are calculated from the cone-beam projection data at point A and point B, respectively. The DPI from the point A is equal to the DPI from the point B if the projection data at the two points A and B are consistent (i.e. no motion). The difference in the two DPI values represents the inconsistency of the two projection data sets from the two views. Assuming that the inconsistency is primarily caused by motion, the difference in the two DPI values defines an amount of motion and is used as a motion index.

The two points A and B shown in FIG. 4 are sufficiently close so that they are apart from each other by an angular range less than 180 degrees apart. Since the point A travels over a short distance on the X-ray source trajectory ST to reach the point B, the associated projection data at the two points A and B have a corresponding amount of short time difference as compared with a case in which the angular interval between the point A and the point B is less than 180 degrees. In other words, the projection data at the two points A and B have a sufficiently appropriate temporal resolution.

There are no specific restrictions on the test plane in the above description, except that the plane should include two points to be compared. In other words, the angle (cone angle) defined by the projection center and the test plane described above is arbitrary. Calculation of DPIs using a middle plane as a test plane will be described. A middle plane is a plane including two points to be compared and having an inclination angle of cone angle $\alpha$ = 0 degree.

FIG. 5 is another diagram illustrating a method of determining a motion index according to this embodiment. The motion index is used for selecting consistent portions of projection data. Cone-beam X-rays are typically emitted from the X-ray source 3 that travels along an X-ray source trajectory ST. As described with respect to FIG. 4, at each projection view, the X-rays are emitted with a cone angle $\alpha$ and a fan angle $\gamma$ and pass through an object o. Although these angles are not shown in FIG. 5, the projection data are acquired by the data acquisition unit 11 in the above described manner.

As shown in FIG. 5, a plane P-4 is a middle plane. The middle plane is a plane that contains the circular X-ray source trajectory ST along points A and B. Assuming a cone-beam scan with a circular trajectory, the middle plane is taken as the test plane from a family of other planes. The DPI in the middle plane is obtained from the projection data near the middle plane. To calculate the derivative, at least two projection data sets are required in each view. The DPI from each view would be identical if the projection data sets were consistent. On the other hand, the variation in the DPI would exist if the projection data sets were inconsistent. In any case, the temporal resolution for any quick or subtle movement is accurately detected using DPIs without involving too much calculation. This is because the two X-ray source 3 locations are at any angle with each other in calculating a difference between the two DPIs. That is, At (time for the X-ray source 3 to move from the first location to the second location) can be made as small as the two adjacent X-ray source 3 views. In addition, test planes are flexibly selected. From Equation (10), the DPI on the middle plane for the circular trajectory is determined by the following Equation (12). This is because the cone angle $\alpha$ is assumed to be 0 for the middle plane.

$$\left.\frac{\partial p(1,\hat{n})}{\partial 1}\right|_{1=\bar{a}(\lambda)\cdot\hat{n}} = -\int_0^{2\pi}\left.\frac{\partial g(\lambda,\alpha,\gamma)}{\partial\alpha}\right|_{\alpha=0}d\gamma \qquad (12)$$

In relation to FIG. 5, a family of other planes may be utilized to analyze complex movements based upon the DPIs associated with the family of planes. For example, cardiac movements are not uniform in all directions. When a plurality of the planes are used as test planes for certain non-uniform cardiac movements, a set of the DPIs associated with a first family of planes may indicate some movement in certain directions while the same DPIs associated with a second family of planes may not indicate any movement in certain other directions.

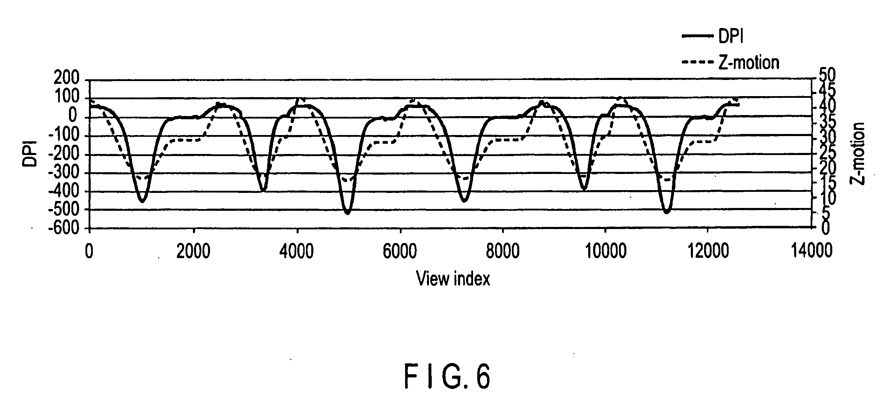

FIG. 6 is a diagram illustrating a numerical simulation utilized to show the accuracy and temporal resolution of DPI. The numerical simulation of a beating heart is based upon a beating hollow ellipsoid having a 30-mm diameter outer wall and a 25-mm diameter inner wall. The center of the ellipsoid is placed at a fixed Z position while the x- and z-dimensions of the ellipsoid expand and contract with time to simulate the beating heart. In order to confirm the effectiveness of the DPI motion index for the cardiac motion, the simulated heart expanded and contracted in an irregular manner from beat to beat by varying period and amplitude. As shown in FIG. 6, the DPI is indicated by a solid line while the motion in the Z-direction of the hart (Z-motion) is indicated by a dotted line. The cardiac motion phases as indicated in the Z-motion substantially correspond to the DPI curve for each heart beat. In other words, the DPI motion index substantially reflects various patterns in cardiac motion.

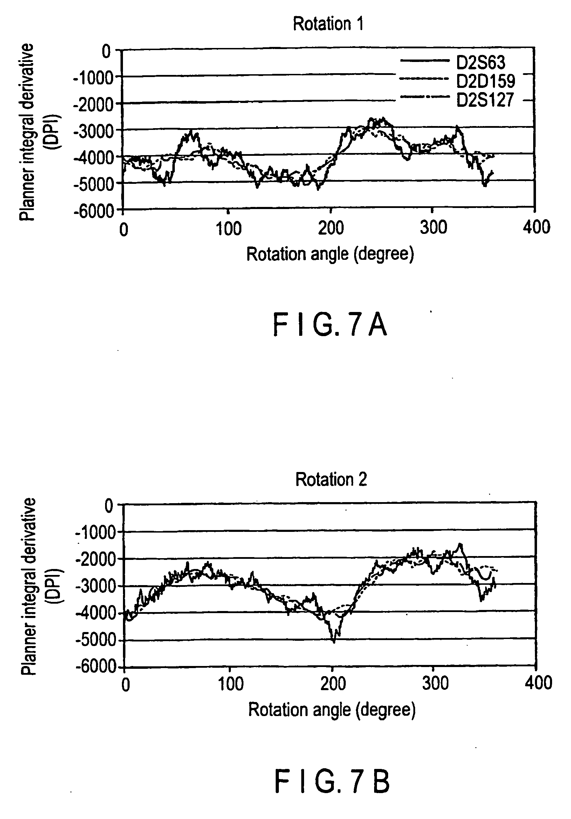

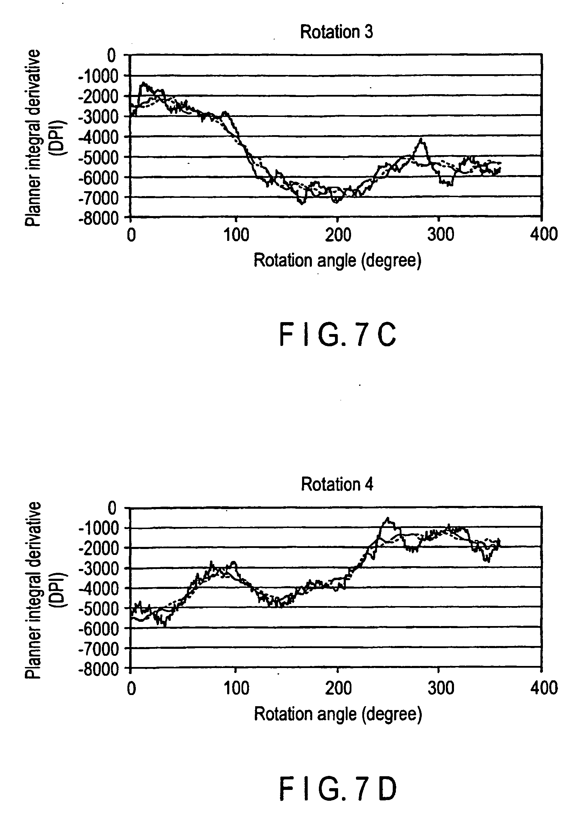

FIGS. 7A, 7B, 7C, and 7D show four consecutive rotations over a circular trajectory in a clinical cardiac scan. For each rotation, the DPIs are averaged with three widths of 63, 127, and 159. It appears that two peaks near 90 degrees and 300 degrees are attributed to beam hardening, which could be misinterpreted as cardiac motion. The above false peaks are more conspicuous in certain combinations of the rotation and the data set than other combinations of the rotation and the data set.

More specifically, referring to FIGS. 7A, 7B, 7C, and 7D, each solid line (D2S63) indicates that the number of terms of the running average of DPI is 63, each dotted line (D2D159) indicates that the number of terms of the running average of DPI is 159, and each one-dot dashed line (D2S127) indicates that the number of terms of the running average of DPI is 127. That is, the value at each point on the solid line (D2S63) is the average value of the DPIs in 63 views centered at the each point, the value at each point on the dotted line (D2D159) is the average value of the DPIs in 159 views centered at the each point, and the value at each point on the one-dot dashed line (D2S127) is the average value of the DPIs in 127 views centered at the each point.

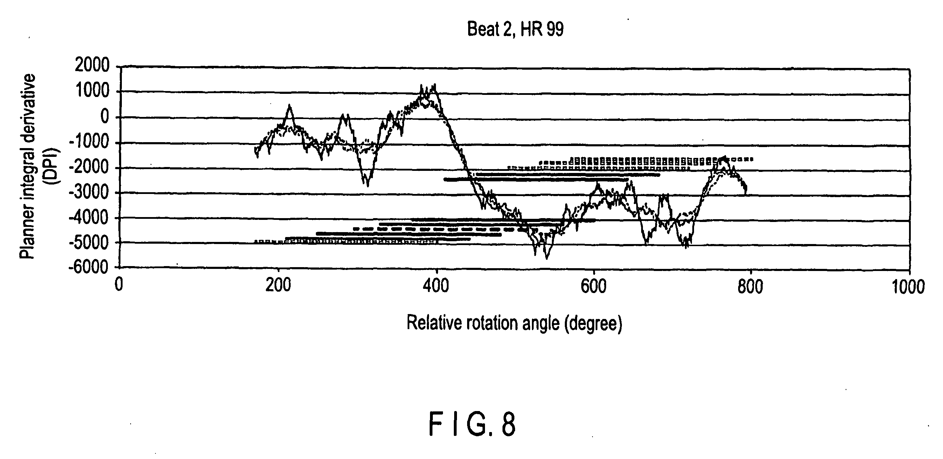

FIG. 8 is a diagram illustrating changes in DPI over the course of one heart beat. As already described with respect to FIG. 7, although there appears to be the similar periodic feature of the beam hardening effect, the DPI values quickly change in response to certain cardiac motions. As shown in FIG. 8, the data acquisition range is defines in the X-axis and covers range over approximately 600 rotation angle degrees. The DPI values at each rotation angle are plotted in the Y-axis. The three sets of data are shown in the graph of FIG. 8. Furthermore, the graph of FIG. 8 includes three types of horizontal bars.

Each bar type indicates a half scan view range that is shifted by 100 views from its preceding range. A dotted double bar represents a view range involving a relatively small amount of cardiac motion while a solid bar or a thick dotted single bar indicates a relatively large amount of cardiac motion. For example, the projection data sets, ranging from approximately 500 degrees to approximately 800 degrees, involve the least amount of cardiac motion. The projection data sets of this angle range except for a single half scan view ranging from approximately 200 degrees to approximately 400 degrees. As indicated by the thick dotted single bar, a single half scan view involves a large amount of motion. But, it is not clear as to why the reconstructed image based on the projection data set belonging to this single half scan view includes less motion artifact components.

In this manner, the processing unit 12 can select projection data belonging to a view range with a relatively small amount of motion from the projection data acquired during an image period by using DPIs. For example, the processing unit 12 calculates a plurality of DPIs based on a plurality of projection data sets belonging to an arbitrary reconstruction range (for example, a half scan view range or a full scan view range) by the above method. The processing unit 12 calculates a motion map based on the plurality of DPIs. If the motion map is less than a predetermined threshold, the processing unit 12 selects projection data belonging to this reconstruction range, and reconstructs an image based on the selected projection data. The reconstructed image does not include many motion artifact components. If the motion map is larger than the preset threshold, projection data belonging to this reconstruction range is inconsistent. Therefore, the image reconstructed based on the projection data belonging to the reconstruction range includes relatively many motion artifacts.

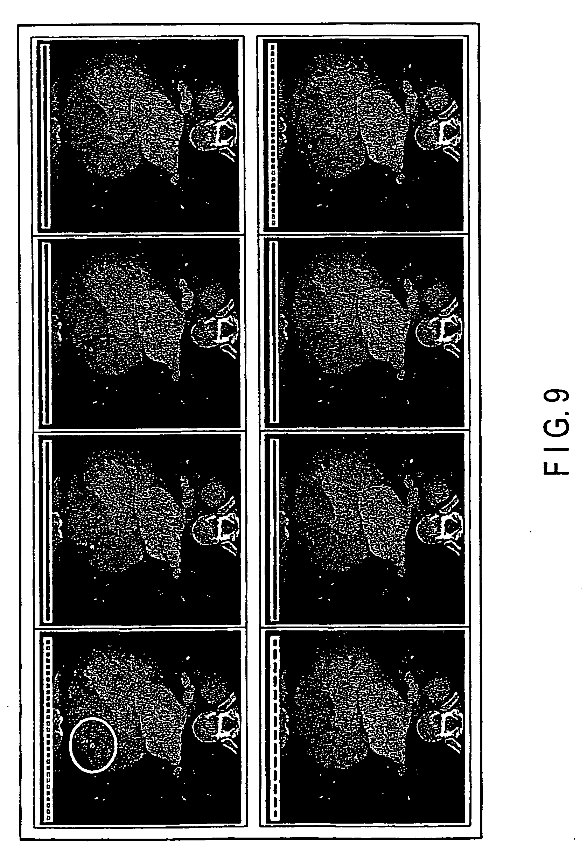

FIG. 9 is a diagram illustrating the reconstructed images based on the selected half scan projection data of FIG. 8. The reconstructed images with a dotted double bar have been reconstructed from the projection data in the dotted double half scan view range of FIG. 8. The dotted double half scan reconstructed images illustrate substantially no motion artifacts near the artery as indicated by a white circle in the top left image of FIG. 9. Obviously, the motion artifacts near the artery are caused by the projection data that has been acquired during the presence of cardiac motion. An exception is the image with a dotted single bar. The images with a dotted single bar have been reconstructed from the projection data in the dotted single half scan view range of FIG. 8. The dotted single half scan reconstructed image illustrates substantially no motion artifacts near the same artery as indicated by the white circle in the top left reconstructed image. Although the image appears to be motion contaminated according to the DPI curve in FIG. 8, the image looks free of motion artifacts.

According to the above description, the medical imaging apparatus is an X-ray CT imaging system. However, the medical imaging apparatus according to this embodiment is not limited to this, and it is also possible to apply the embodiment to a PET (positron emission tomography) imaging system and SPECT (single photon emission computed tomography) imaging system. The PET imaging system and SPECT imaging system according to the embodiment will be sequentially described below.

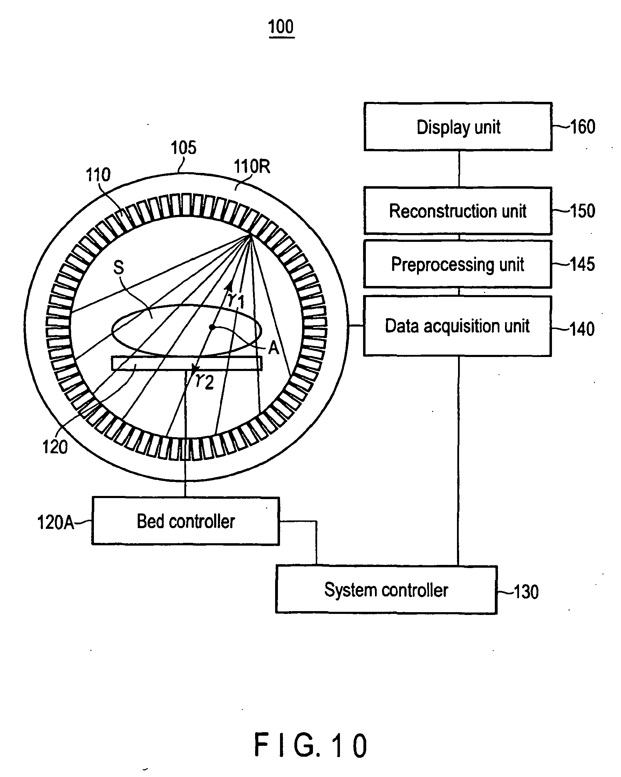

FIG. 10 is a diagram illustrating the arrangement of a positron emission tomography (PET) imaging system 100 according to this embodiment. The PET imaging system 100 includes a PET detector unit 105. The PET detector unit 105 accommodates a plurality of two-dimensional gamma ray detectors 110. The gamma ray detectors 110 are installed on a detector ring 110R around an object S. The object S is laid on a sliding bed 120. A bed controller 120A controls the sliding movement, of the sliding bed 120, along a direction normal to a predetermined plane defined by the gamma ray detectors 110. The detector ring 110R is stationary while the sliding bed 120 moves along the central axis of the detector rings 110R at a predetermined speed. That is, the detector rings 110R are stationary during a data acquisition process.

A data acquisition unit 140 controls the plurality of gamma ray detectors 110 and receives the gamma photon data that are detected at the gamma ray detectors 110. Unlike an X-ray CT system, the PET imaging system 100 acquires the emission data rather than the projection data. That is, the PET imaging is based upon the gamma photons that are ultimately emitted inside the object of the interest due to the tracer isotope. Thus, a preprocessing unit 145 preprocesses the emission data before reconstruction by converting the emission data to projection data. Furthermore, the preprocessing unit 145 determines motion index values for the projection data using the DPI according to this embodiment. In addition, the preprocessing unit 145 selects projection data based upon the motion index values so that the selected projection data portions reflect a minimal amount of motion during the data acquisition. The reconstruction unit 150 reconstructs a plurality of images based upon the selected projection data. A display unit 160 displays the plurality of images with a minimal amount of motion artifacts. A system controller 130 in turn controls both the bed controller 120A and the data acquisition unit 140. In the embodiment, emission data are acquired by the above method while the sliding bed 120 travels.

The PET imaging system 100 operates in a manner that PET imaging is based upon the tracer isotope concentration distribution. In this regard, before a PET scan, tracer isotope is chemically incorporated into biologically active molecules such as fluorodeoxyglucose (FDG). After a waiting period during which the active molecules become concentrated in tissues of interest, the PET imaging system 100 acquires raw data from the patient as the tracer isotope decays. In further detail, during position emission decay, the tracer isotope emits a positron. The positron travels in tissue for a short distance as it loses kinetic energy until it interacts with an electron. Consequently, the positron annihilates and emits two gamma photons with energy of 511 keV in approximately 180 degrees apart in the opposite directions. Ultimately, the two gamma ray photons can be almost simultaneously detected by the gamma ray detectors 110.

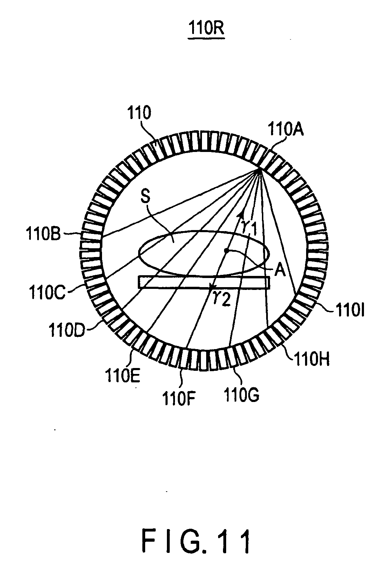

FIG. 11 is a diagram illustrating detector rings 110R included in the PET imaging system 100. The PET detector unit 105 is constituted of the plurality of detector rings 110R to cover a long object such as a human. Each detector ring 110R is built by many units of gamma ray detectors 110 such as photomultiplier tubes or silicon avalanche photodiodes (Si APD). The raw data are acquired by pairs of the gamma ray detectors 110 and are a list of coincidence events representing near-simultaneous detection of annihilation photons. That is, each coincidence event defines a line in space connecting the two gamma ray detectors 110 along which the positron emission occurred. For example, from a certain point A in an object S emits two photons along a direction of $\gamma_1$ towards a gamma ray detector 110A and another direction of $\gamma_2$ towards a gamma ray detector 110F. These two photons travel approximately 180 degrees with each other in the opposite directions as indicated by the two arrows. FIG. 11 also illustrates that other points in the same object emit photons in substantially the same manner. That is, other pairs of the gamma ray detectors such as 110A/110C, 110A/110D, 110A/110E, 110A/110G and 110A/110H also sequentially detect annihilation photons near-simultaneously. Emission data are preprocessed prior to a reconstruction process.

On the preprocessing unit 145 according to this embodiment, the coincidence events are grouped into projection images such as sinograms based upon the angle of each view and tilt. In other words, the sinogram images are analogous to the projections captured by CT scanners. Although the sinogram images are reconstructed in a similar way as CT, since a normal PET data set has millions of counts for the whole acquisition while the CT can reach a few billion counts, the PET data suffer from artifacts such as scatter and random events much more dramatically than the CT data. For the above reasons, a considerable amount of pre-processing is required for the PET data and includes correction for random coincidences, correction for estimation and subtraction of scattered photons, detector dead-time correction, and detector-sensitivity correction.

In addition, the preprocessing unit 145 selects a minimal motion portion of the projection data. The integrated coincidental signals are expressed in Equation (13) below:

$$g(z,\phi;z',\phi') \approx \int_l dl\, a(\bar{x}) \exp\left[-\int_l dl'\, \mu(\bar{x})\right] \qquad (13)$$

where $(z,\phi)$ and $(z',\phi')$ indicate the locations of the two detectors. $a(\bar{x})$ is the tracer isotope concentration. $\mu(\bar{x})$ represents the attenuation map. The integrals are executed along the line determined by the two detectors.

Assuming that the attenuation map is known, the corrected data are expressed in Equation (14) below:

$$g_c(z, \phi; z', \phi') = g(z, \phi; z', \phi') \exp\left[ + \int_l dl' \mu(\bar{x}) \right] = \int_l dl\, a(\bar{x}) \qquad (14)$$

Fixing one detector unit, the corrected data are cone-beam projections of the tracer isotope concentration a (x-). The derivatives of the planar integral (DPI) of a(x-) are calculated from the corrected data $g_c$ (z,$\phi$; z',$\phi$'). Based upon the DPIs, the preprocessing unit 145 selects a minimal motion portion of the projection data.

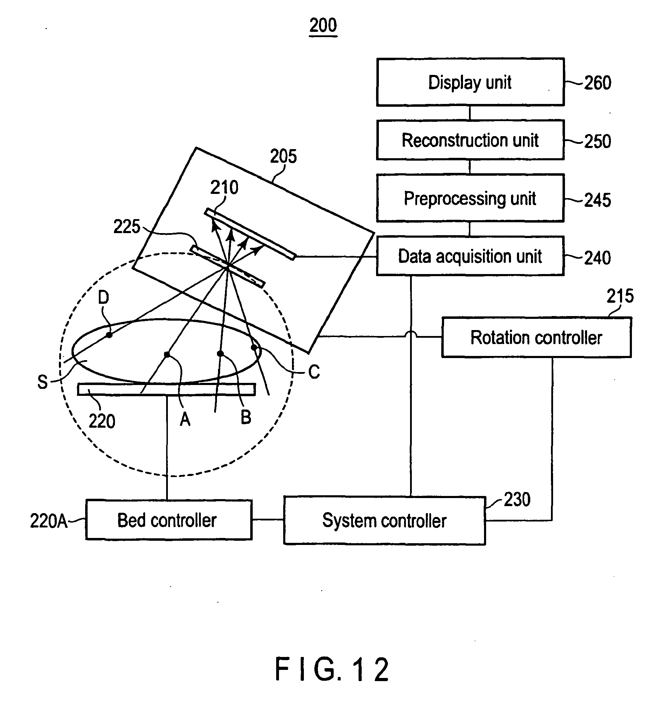

FIG. 12 is a diagram illustrating a SPECT imaging system 200 according to this embodiment. The SPECT imaging system 200 includes at least one gamma ray detection unit (gamma camera) 205. The gamma ray detection unit accommodates a two-dimensional gamma ray detector 210. The gamma camera 205 is placed over an object S. The object S is laid on a sliding bed 220. A rotational controller 215 rotates the gamma ray detector 205 around the object S as indicated by the dotted circle. A bed controller 220A controls the sliding movement of the sliding bed 220. The gamma camera 205 rotates around the object S while the sliding bed 220 moves along the central axis of the rotation at a predetermined speed during a data acquisition process.

A data acquisition unit 240 controls the gamma ray detector 210 and receives the emission data that are detected at the gamma ray detector 210. Unlike an X-ray CT imaging system, the SPECT imaging system 200 acquires the gamma ray photon data rather than the projection data. That is, the SPECT imaging is based upon the gamma ray photon that is directly emitted inside the object of the interest due to the tracer isotope. A preprocessing unit 245 preprocesses the emission data before reconstruction by converting the emission data to projection data. Furthermore, the preprocessing unit 245 determines motion index values for the projection data using the derivatives of the planar integrals (DPI) according to this embodiment. The preprocessing unit 245 selects projection data based upon the motion index values so that the selected projection data portions reflect a minimal amount of motion during the data acquisition. A reconstruction unit 250 reconstructs a plurality of images based on the selected projection data. A display unit 260 displays the plurality of images with a minimal amount of motion artifacts. A system controller 230 in turn controls both the bed controller 220A and the data acquisition unit 240. In the embodiment, emission data are acquired in the following manner while the sliding bed 220 travels.

The SPECT imaging system 200 operates in a manner that SPECT imaging is based upon the tracer isotope concentration distribution. In this regard, before a SPECT scan, tracer isotope that emits gamma radiation is injected into the patient body. The SPECT tracer isotopes are generally longer half-life more easily-obtained isotopes than the PET tracer isotopes. A tracer isotope having its radioactive properties has been attached to a special radioligand having its chemical properties that allow specifically binding to certain types of tissues. The SPECT imaging system 200 acquires raw data from the patient as the tracer isotope emits gamma radiation.

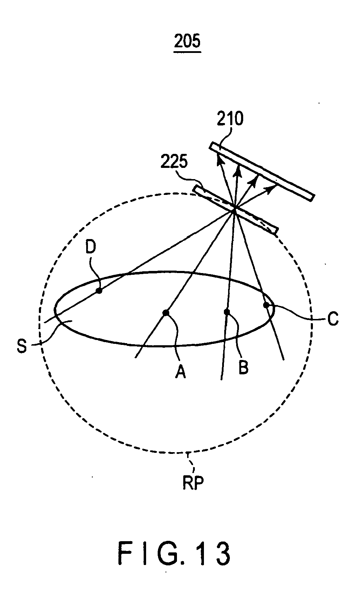

Now referring to FIG. 13, the gamma camera 205 directly measures gamma radiation emitted from a region of interest in the object S. In the illustrated region of interest, four locations A, B, C and D emit gamma rays as indicated by the corresponding arrows. When the gamma camera 205 is located at a certain position on a rotational path RP as indicated by a dotted line, the two-dimensional array detector 210 captures the raw data of the gamma rays that pass through a pinhole 225. That is, selected gamma rays are detected, and the data are acquired. For example, from a certain point A in an object S emits gamma rays in different directions, but a gamma ray that travels through the pinhole 225 is recorded as indicated by the arrow. FIG. 13 also illustrates that other points in the same object emit gamma rays and they are recorded in substantially the same manner as the gamma camera 205 rotates along the predetermined rotational path RP. These emission data are preprocessed prior to a reconstruction process.

According to this embodiment of the preprocessing unit 245, the gamma ray events are grouped into projection images such as sinograms based upon the angle of each view and tilt. In other words, the sinogram images are analogous to the projections captured by CT scans. As in the case of PET, a considerable amount of pre-processing is required for the PET data and includes correction for estimation, correction for subtraction of scattered photons, detector dead-time correction and detector-sensitivity correction.

In the pinhole SPECT imaging system 200, the measured signals on the 2D array detector are expressed in Equation (15):

$$g(\bar{x}_0, u, v) \approx \int_0^\infty dt\, a(\bar{x}_0 + t\hat{e}) \exp\left[ - \int_0^\infty dt' \mu(\bar{x}_0 + t\hat{e} + t'\hat{e}) \right] \qquad (15)$$

where $x_0$- indicates the pinhole location; (u,v) is a position on the 2D detector; a($\cdot$) represents the tracer concentration; and $\mu(\cdot)$ is the attenuation map. Unit vector e^ is determined by the pinhole location $x_0$- and the detector point (u,v). If the attenuation is ignored for the purpose of approximation, the projection data in a SPECT scan is expressed as in

Equation (16):

$$g(\bar{x}_0, u, v) \approx \int_0^\infty dt\, a(\bar{x}_0 + t\hat{e}) \qquad (16)$$

Equation (16) is a cone-beam transform of the tracer concentration. Thus, Grangeat's formula is used to obtain the DPI of a(·) for the above described SPECT data. The preprocessing unit 245 selects a minimal motion portion of the projection data by using the DPIs.

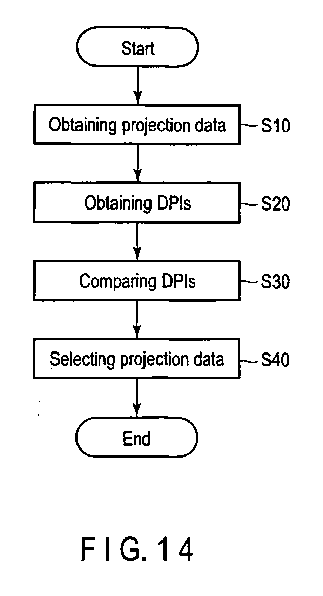

FIG. 14 shows a typical procedure for the processing of selecting a consistent portion of projection data for reconstructing images with substantially minimal amount of movement according to this embodiment. In the processing according to the embodiment, first of all, in step S10, this system acquires projection data associated with a region of interest in an object. For example, the projection data is directly acquired from an X-ray CT scanner. In another example, the projection data is indirectly acquired from a PET scanner based upon the emission data. In step S20, the DPIs are calculated from the projection data. For CT, the DPI is determined from projection data according to Equation (10). For PET, the DPI of a(x⁻) are calculated from the corrected data as described in Equation (14). For SPECT, Grangeat's formula is used to obtain the DPI of a(·) from the SPECT data as described in Equation (16). In step S30, the DPIs are compared to determine an amount of movement of the region of interest based upon the difference between the DPIs. That is, the smaller the difference, the less the region of interest has moved. In step S40, a portion of the projection data is selected based upon the comparison result in step S30 so that the processing unit 12 ultimately reconstructs an image with substantially minimal movement artifacts.

According to the above description, the medical imaging apparatus according to this embodiment includes at least the data acquisition unit 11, 140 or 240 and the processing unit 12, 145 or 245. The data acquisition unit 11, 140 or 240 acquires first raw data about an object associated with a first position, and second raw data about the object associated with a second position. For example, in the case of the X-ray CT imaging system, "position" means the position of the X-ray source, and "raw data" means projection data.

The processing unit 12, 145 or 245 evaluates the amount of motion of the object between the acquisition time of the first raw data and the acquisition time of the second raw data. More specifically, the processing unit 12, 145 or 245 calculates a first DPI on the test plane including the first and second positions by using the first raw data, and calculates a second DPI on the test plane by using the second raw data. The processing unit 12, 145 or 245 then evaluates the amount of motion by using the difference between the first and second DPIs.

More specifically, the processing unit 12, 145 or 245 calculates a first set of a plurality of integral values at the first position based on the first raw data, and calculates a second set of a plurality of integral values at the second position based on the second raw data. The processing unit 12, 145 or 245 calculates a first plane integral based on the first set of the plurality of integral values, and calculates a second plane integral based on the second set of the plurality of integral values. The processing unit 12, 145 or 245 calculates the first DPI by using the first plane integral, and calculates the second DPI by using the second plane integral. The processing unit 12, 145 or 245 then calculates the difference between the first and second DPIs.

DPIs and DPI differences are used as novel motion indices. These novel motion indices are based on Grangeat's formula, and allow to detect body motion mathematically strictly. Motion indices according to this embodiment are used to identify a consistent portion of projection data. The processing unit reconstructs an image based on the identified consistent portion. The reconstructed image does not include many motion artifacts. The image whose image quality has been improved in this manner prevents the doctor from making inaccurate diagnosis. In addition, motion indices according to the embodiment may be used for, for example, the positioning of an object. Using motion indices according to the embodiment can detect motion with high temporal resolution. This makes it possible to accurately position the object. In addition, motion indices according to the embodiment can be used in various aspects associated with motion evaluation.

According to this embodiment, it is possible to provide a medical imaging apparatus and medical imaging method which can accurately detect the amount of motion of an object.

While certain embodiments have been described, these embodiments have been presented by way of example only, and are not intended to limit the scope of the invention. Indeed, the novel methods and systems described herein may be embodied in a variety of other forms; furthermore, various omissions, substitutions and changes in the form of the methods and systems described herein may be made without departing from the scope of the invention. The accompanying claims and their equivalents are intended to cover such forms or modifications.

**Claims**

1. A medical imaging apparatus comprising:

a data acquisition unit (11, 140, 240) configured to acquire first raw projection data about an object at a first position and second raw projection data about the object at a second position; and
a processing unit (12, 145, 245) configured to evaluate an amount of motion of the object between an acquisition time of the first raw data and an acquisition time of the second raw data, the processing unit (12, 145, 245) calculating a derivative of first plane integrals on a test plane including the first position and the second position by using the first raw data, calculating a derivative of second plane integrals on the test plane by using the second raw data, and evaluating the amount of motion by using a difference between the derivate of the first plane integrals and the derivate of the second plane integrals.

2. The apparatus of claim 1, further comprising:

an X-ray source (3) configured to irradiate the object with X-rays and move along a predetermined trajectory including the first position and the second position; and
an X-ray detector (5) configured to be placed to face the X-ray source (3) and detect X-rays emitted from the X-ray source and transmitted through the object,
wherein the data acquisition unit (11) acquires the first raw data originating from X-rays emitted by the X-ray source (3) from the first position through the X-ray detector (5), and acquires the second raw data originating from X-rays emitted by the X-ray source (3) from the second position through the X-ray detector (5).

3. The apparatus of claim 2, wherein the X-ray source (3) emits cone-beam X-rays.

4. The apparatus of claim 3, wherein the test plane is determined in accordance with a preset cone angle of the cone-beam.

5. The apparatus of claim 4, wherein the cone angle is zero.

6. The apparatus of claim 1, wherein the first position and the second position are arranged at an arbitrary angle on the predetermined trajectory.

7. The apparatus of claim 1, wherein the first position and the second position are spaced apart from each other by 180 degrees on the predetermined trajectory.

8. The apparatus of claim 2, wherein the predetermined trajectory has a circular shape.

9. The apparatus of claim 2, wherein the predetermined trajectory has a helical shape.

10. The apparatus of claim 1, wherein the processing unit (12, 145, 245) calculates a plurality of first line integrals at the first position based on the first raw data, calculates a plurality of second line integrals at the second position based on the second raw data, calculates a first plane integral on the test plane based on the plurality of first line integrals, and calculates a second plane integral on the test plane based on the plurality of second line integrals, and the plurality of first line integrals and the plurality of second line integrals each include a predetermined number of spatially continuous views.

11. The apparatus of claim 1, wherein derivatives of a plurality of plane integrals are used to analyze direction-dependent motion of a plurality of planes.

12. The apparatus of claim 1, further comprising:

an X-ray source (3) configured to irradiate the object with X-rays and move along a predetermined trajectory including the first position and the second position; and
an X-ray detector (5) configured to be placed to face the.X-ray source (3) and detect X-rays emitted from the X-ray source (3) and transmitted through the object,
wherein the data acquisition unit (11) acquires the third raw data originating from X-rays emitted by the X-ray source (3) from the first position through the X-ray detector (5), and acquires the fourth raw data originating from X-rays emitted by the X-ray source (3) from the second position through the X-ray detector (5), and the processing unit (12) calculates a derivative of a third plane integral on the test plane including the first position and the second position by using the third raw data, calculates a derivative of the fourth plane integral on the test plane by using the fourth raw data, and evaluates a second motion amount of the object between an acquisition time of the third raw data and an acquisition time of the fourth raw data by using a difference between the derivative of the third plane integral and the derivative of the fourth plane integral.

13. The apparatus of claim 12, wherein the derivative of the plane integral has direction dependence of motion of the object.

14. The apparatus of claim 1, wherein a smallest amount of motion of the object is selected.

15. The apparatus of claim 1, wherein the first amount of movement is compared with a predetermined value.

16. The apparatus of claim 1, wherein the data acquisition unit (140) acquires gamma photon data as the raw data, acquires first gamma photon data from a predetermined position of the object at the first position, and acquires second gamma photon data from a predetermined position of the object at the second position, and the second position is spaced apart from the first position by substantially 180 degrees.

17. The apparatus of claim 1, wherein the data acquisition unit (240) acquires gamma ray data as the raw data, acquires the first gamma ray data from the object at the first position, and acquires second gamma ray data from the object at the second position.

18. A medical imaging method comprising:

acquiring first raw projection data of the object at a first position by using one of a CT scanner, a PET scanner, and a SPECT scanner;
acquiring second raw projection data of the object at a second position by using one of the CT scanner, the PET scanner, and the SPECT scanner;
calculating a derivative of a first plane integral on a test plane including the first position and the second position by using the first raw data;
calculating a derivative of a second plane integral on the test plane by using the second raw data; and
evaluating an amount of motion of the object between an acquisition time of the first raw data and an acquisition time of the second raw data by using a difference between the derivative of the first plane integral and the derivative of the second plane integral.


**Patentansprüche**

1. Medizinische Bildgebungsvorrichtung, enthaltend:

eine Datenerfassungseinheit (11, 140, 240), die konfiguriert ist zum Erfassen von ersten Projektionsrohdaten über ein Objekt an einer ersten Position und von zweiten Projektionsrohdaten über das Objekt an einer zweiten Position; und
eine Vorarbeitungseiheit (12, 145, 245), die konfiguriert ist zum Evaluieren eines Bewegungsausmaßes des Objekts zwischen einem Erfassungszeitpunkt der ersten Rohdaten und einem Erfassungszeitpunkt der zweiten Rohdaten, wobei die Verarbeitungseinheit (12, 145, 245) eine Ableitung von ersten Flächenintegralen für eine Testebene, die die erste Position und die zweite Position enthält berechnet, indem die ersten Rohdaten verwendet werden, eine Ableitung von zweiten Flächenintegralen für die Testebene berechnet, indem die zweiten Rohdaten verwendet werden, und das Ausmaß der Bewegung evaluiert, indem eine Differenz verwendet wird zwischen der Ableitung der ersten Flächenintegrale und der Ableitung der zweiten Flächenintegrale.

2. Vorrichtung nach Anspruch 1, ferner enthaltend:

eine Röntgenquelle (3), die konfiguriert ist zum Bestrahlen des Objekts mit Röntgenstrahlen und zum Bewegung entlang einer vorbestimmten Bewegungsbahn, die die erste Position und die zweite Position enthält; und
einen Röntgendetektor (5), der konfiguriert ist, um platziert zu werden, um der Röntgenquelle (3) gegenüber zu liegen und Röntgenstrahlen, die von der Röntgenquelle ausgesendet und durch das Objekt übertragen werden, zu detektieren,

wobei die Datenerfassungseinheit (11) die ersten Rohdaten erfasst, die von den Röntgenstrahlen stammen, die von der Röntgenquelle (3) emittiert werden, von der ersten Position durch den Röntgendetektor (5), und die zweiten Rohdaten erfasst, die von Röntgenstrahlen stammen, die von den Röntgenquelle (3) emittiert werden, von der zweiten Position durch den Röntgendetektor (5).

3. Vorrichtung nach Anspruch 2, bei der die Röntgenquelle (3) kegelstrahlformige Röntgenstrahlen emittiert.

4. Vorrichtung nach Anspruch 3, bei der die Testebene bestimmt wird gemäß einem vorgesetzten Kegelwinkel des Kegelstrahls.

5. Vorrichtung nach Anspruch 4, bei der der Kegelwinkel Null ist.

6. Vorrichtung nach Anspruch 1, bei der die erste Position und die zweite Position bei einem willkürlichen Winkel auf der vorbestimmten Bewegungsbahn angeordnet sind.

7. Vorrichtung nach Anspruch 1, bei der die erste Position und die zweite Position voneinander um 180 Grad von der vorbestimmten Bewegungsbahn beabstandet sind.

8. Vorrichtung nach Anspruch 2, bei der die vorbestimmte Bewebungsbahn eine Kreisform hat.

9. Vorrichtung nach Anspruch 2, bei der die vorbestimmte Bewegungsbahn eine Spiralform hat.

10. Vorrichtung nach Anspruch 1, bei der die Verarbeitungseinheit (12, 145, 245) eine Mehrzahl von ersten Kurvenintegralen an der ersten Position basierend auf den ersten Rohdaten berechnet, eine Mehrzahl von zweiten Kurvenintegralen an der zweiten Position basierend auf den zweiten Rohdaten berechnet, ein erstes Flächenintegral für die Testebene basierend auf der Mehrzahl von ersten Kurvenintegralen berechnet, und ein zweites Flächenintegral für die Testebene basierend auf der Mehrzahl von zweiten Kurvenintegralen berechnet, und die Mehrzahl von ersten Kurvenintegralen und die Mehrzahl von zweiten Kuzvenintegralen jeweils eine vorbestimmte Anzahl von räumlich fortlaufenden Ansichten enthalten.

11. Vorrichtung nach Anspruch 1, bei der die Ableitungen von einer Mehrzahl von Flachenintegralen verwendet werden, um eine richtungsabhängige Bewegung einer Mehrzahl von Ebenen zu analysieren.

12. Vorrichtung nach Anspruch 1, ferner enthaltend:

eine Röntgenquelle (3), die konfiguriert ist zum Bestrahlen des Objekts mit Röntgenstrahlen und zum Bewegung entlang einer vorbestimmten Bewegungsbahn, die die erste Position und die zweite Position enthält, und einen Röntgendetektor (5), der konfiguriert ist, um platziert zu sein, um der Röntgenquelle (3) gegenüber zu liegen und Röntgenstrahlen zu detektieren, die von der Röntgenquelle (3) emittiert und durch das Objekt übertragen werden,

wobei die Datenerfassungseinheit (11) die dritten Rohdaten erfasst, die von Röntgenstrahlen stammen, die von der Röntgenquelle (3) emittiert werden, von der ersten Position durch den Röntgendetektor (5), und die vierten Rohdaten erfasst, die von Röntgenstrahlen stammen, die von der Röntgenquelle (3) emittiert werden, von der zweiten Position durch den Röntgendetektor (5), und

die Verarbeitungseinheit (12) eine Ableitung eines dritten Flächenintegrals für die Testebene, die die erste Position und die zweite Position enthält, berechnet, indem die dritten Rohdaten, verwendet werden, eine Ableitung des vierten Flächenintegrals für die Testebene berechnet, indem die vierten Rohdaten verwendet werden, und eines zweiten Bewegungsausmaßes des Objekts evaluiert zwischen einem Erfassungszeitpunkt der dritten Rohdaten und einem Erfassungszeitpunkt der vierten Rohdaten, indem eine Differenz verwendet wird zwischen der Ableitung des dritten Flächenintegrals und der Ableitung des vierten Flächenintegrals,

13. Vorrichtung nach Anspruch 12, bei der die Ableitung des Flächenintegrals eine Richtungsabhängigkeit von der Bewegung des Objekts hat.

14. Vorrichtung nach Anspruch 1, bei der ein kleinstes Ausmaß an Bewegung des Objekts ausgewählt wird.

15. Vorrichtung nach Anspruch 1, bei der das erste Ausmaß an Bewegung mit einem vorbestimmten Wert verglichen wird.

**16.** Vorrichtung nach Anspruch 1, bei der die Datenerfassungseinheit (140) Gammaphotonendaten als die Rohdaten erfasst, erste Gammaphotonendaten von einer vorbestimmten Position des Objekts an der ersten Position erfasst, und zweite Gammaphotonendaten von einer vorbestimmten Position des Objekts an der zweiten Position erfasst, und die zweite Position von der ersten Position im Wesentlichen um 180 Grad beabstandet ist

**17.** Vorrichtung nach Anspruch 1, bei der die Datenerfassungseinheit (240) Gammarohdaten als die Rohdaten erfasst, die ersten Gammarohdaten von dem Objekt an der ersten Position erfasst, und zweite Gammarohdaten von dem Objekt an der zweiten Position erfasst.

**18.** Medizinisches Bildgebungsverfahren, enthaltend:

Erfassen von ersten Projektionsrohdaten des Objekts an einer ersten Position, indem ein CT-Scanner, ein PET-Scanner oder ein SPECT-Scanner verwendet wird;
Erfassen von zweiten Projektionsrohdaten des Objekts an einer zweiten Position, indem der CT-Scanner, der PET-Scanner oder SPECT-Scanner verwendet wird;
Berechnen einer Ableitung eines ersten Flächenintegrals für eine Testebene, die die erste Position und die zweite Position enthält, indem die ersten Rohdaten verwendet werden;
Berechnen einer Ableitung eines zweiten Flächenintegrals für die Testebene, indem die zweiten Rohdaten verwendet werden; und
Evaluieren eines Ausmaßes an Bewegung des Objekts zwischen einem Erfassungszeitpunkt der ersten Rohdaten und einem Erfassungszeitpunkt der zweiten Rohdaten, indem eine Differenz verwendet wird zwischen der Ableitung des ersten Flächenintegrals und der Ableitung des zweiten Flächenintegrals.

## Revendications

**1.** Appareil d'imagerie médicale comprenant :

une unité d'acquisition de données (11, 140, 240) configurée pour acquérir des premières données de projection brutes concernant un objet à une première position et des deuxièmes données de projection brutes concernant l'objet à une deuxième position ; et
une unité de traitement (12, 145, 245) configurée pour évaluer une quantité de mouvement de l'objet entre un instant d'acquisition des premières données brutes et un instant d'acquisition des deuxièmes données brutes, l'unité de traitement (12, 145, 245) calculant une dérivée de premières intégrales dans le plan dans un plan de test comprenant la première position et la deuxième position en utilisant les premières données brutes, calculant une dérivée de deuxièmes intégrales dans le plan dans le plan de test en utilisant les deuxièmes données brutes, et évaluant la quantité de mouvement en utilisant une différence entre la dérivée des premières intégrales dans le plan et la dérivée des deuxièmes intégrales dans le plan.

**2.** Appareil selon la revendication 1, comprenant en outre :

une source de rayons X (3) configurée pour irradier l'objet avec des rayons X et se déplacer le long d'une trajectoire prédéterminée comprenant la première position et la deuxième position ; et
un détecteur de rayons X (5) configuré pour être placé de manière à faire face à la source de rayons X (3) et détecter les rayons X émis par la source de rayons X et transmis à travers l'objet,
dans lequel l'unité d'acquisition de données (11) acquiert les premières données brutes provenant des rayons X émis par la source de rayons X (3) à partir de la première position par l'intermédiaire du détecteur de rayons X (5), et acquiert les deuxièmes données brutes provenant des rayons X émis par la source de rayons X (3) à partir de la deuxième position par l'intermédiaire du détecteur de rayons X (5).

**3.** Appareil selon la revendication 2, dans lequel la source de rayons X (3) émet des rayons X à faisceau conique.

**4.** Appareil selon la revendication 3, dans lequel le plan de test est déterminé selon un angle de cône présélectionné du faisceau conique.

**5.** Appareil selon la revendication 4, dans lequel l'angle de cône est nul.

**6.** appareil selon la revendication 1, dans lequel la première position et la deuxième position sont agencées selon un angle arbitraire sur la trajectoire prédéterminée.

7. Appareil selon la revendication 1, dans lequel la première position et la deuxième position sont espacées l'une de l'autre de 180 degrés sur la trajectoire prédéterminée.

8. Appareil selon la revendication 2, dans lequel la trajectoire prédéterminée a une forme circulaire.

9. Appareil selon la revendication 2, dans lequel la trajectoire prédéterminée a une Forme hélicoïdale.

10. Appareil selon la revendication 1, dans lequel l'unité de traitement (12, 145, 245) calcule une pluralité de premières intégrales curvilignes à la première position sur la base des premières données brutes, calcule une pluralité de deuxièmes intégrales curvilignes à la deuxième position sur la base des deuxièmes données brutes, calcule une première intégrale dans le plan dans le plan de test sur la base de la pluralité de premières intégrales curvilignes, et calcule une deuxième intégrale dans le plan dans le plan de test sur la base de la pluralité de deuxièmes intégrales curvilignes, et
la pluralité de premières intégrales curvilignes et la pluralité de deuxièmes intégrales curvilignes comprennent chacune un nombre prédéterminé de vues spatialement continues.

11. Appareil selon la revendication 1, dans lequel les dérivées d'une pluralité d'intégrales dans le plan sont utilisées pour analyser un mouvement dépendant de la direction d'une pluralité de plans.

12. Appareil selon la revendication 1, comprenant en outre :

une source de rayons X (3) configurée pour irradier l'objet avec des rayons X et se déplacer le long d'une trajectoire prédéterminée comprenant la première position et la deuxième position ; et
un détecteur de rayons X (5) configuré pour être placé de manière à faire face à la source de rayons X (3) et détecter les rayons X émis par la source de rayons X (3) et transmis à travers l'objet,
dans lequel l'unité d'acquisition de données (11) acquiert les troisièmes données brutes provenant des rayons X émis par la source de rayons X (3) à partir de la première position par l'intermédiaire du détecteur de rayons X (5), et acquiert les quatrièmes données brutes provenant des rayons X émis par la source de rayons X (3) à partir de la deuxième position par l'intermédiaire du détecteur de rayons X (5), et
l'unité de traitement (12) calcule une dérivée d'une troisième intégrale dans le plan dans le plan de test comprenant la première position et la deuxième position en utilisant les troisièmes données brutes, calcule une dérivée de la quatrième intégrale dans le plan dans le plan de test en utilisant les quatrièmes données brutes, et évalue une deuxième quantité de mouvement de l'objet entre un instant d'acquisition des troisièmes données brutes et un instant d'acquisition des quatrièmes données brutes en utilisant une différence entre la dérivée de la troisième intégrale dans le plan et la dérivée de la quatrième intégrale dans le plan.

13. Appareil selon la revendication 12, dans lequel la dérivée de l'intégrale dans le plan dépend de la direction de mouvement de l'objet.

14. Appareil selon la revendication 1, dans lequel une quantité la plus faible de mouvement de l'objet est sélectionnée.

15. Appareil selon la revendication 1, dans lequel la première quantité de mouvement est comparée à une valeur prédéterminée.

16. Appareil selon la revendication 1, dans lequel l'unité d'acquisition de données (140) acquiert des données de photons gamma en tant que données brutes, acquiert des premières données de photons gamma d'une position prédéterminée de l'objet à la première position, et acquiert des deuxièmes données de photons gamma d'une position prédéterminée de l'objet à la deuxième position, et la deuxième position est espacée de la première position sensiblement de 180 degrés.

17. Appareil selon la revendication 1, dans lequel l'unité d'acquisition de données (240) acquiert des données de rayons gamma en tant que données brutes, acquiert les premières données de rayons gamma provenant de l'objet à la première position, et acquiert les deuxièmes données de rayons gamma provenant de l'objet à la deuxième position.

18. Procédé d'imagerie médicale comprenant :

l'acquisition de premières données de projection brutes de l'objet à une première position en utilisant l'un d'un scanner de tomodensitométrie, d'un scanner de tomographie d'émission de positons et d'un scanner de tomographie d'émission monophotonique ;

l'acquisition de deuxièmes données de projection brutes de l'objet à une deuxième position en utilisant l'un du scanner de tomodensitométrie, du scanner de tomographie d'émission de positons et du scanner de tomographie d'émission monophotonique ;

le calcul d'une dérivée d'une première intégrale dans le plan dans un plan de test comprenant la première position et la deuxième position en utilisant les première données brutes ;

le calcul d'une dérivée d'une deuxième intégrale dans le plan dans le plan de test en utilisant les deuxièmes données brutes ; et

l'évaluation d'une quantité de mouvement de l'objet entre un instant d'acquisition des premières données brutes et un instant d'acquisition des deuxièmes données brutes en utilisant une différence entre la dérivée de la première intégrale dans le plan et la dérivée de la deuxième intégrale dans le plan.

F I G. 1

FIG. 2

FIG. 3

FIG. 4

FIG. 5

F I G. 6

Rotation 1

F I G. 7 A

Rotation 2

F I G. 7 B

Rotation 3

F I G. 7 C

Rotation 4

F I G. 7 D

Beat 2, HR 99

FIG. 8

EP 2 490 180 B1

FIG.9

100

Display unit ~160

Reconstruction unit ~150

Preprocessing unit ~145

Data acquisition unit ~140

Bed controller

120A~

System controller ~130

F I G. 1 0

<u>110R</u>

F I G. 1 1

<u>200</u>

| | |
|---|---|
| Display unit | ~260 |
| Reconstruction unit | ~250 |
| Preprocessing unit | ~245 |
| Data acquisition unit | ~240 |

Rotation controller ~215

Bed controller

System controller ~230

220A

205

210

225

D

S

220

A    B

C

# FIG. 12

FIG. 13

Start

Obtaining projection data — S10

Obtaining DPIs — S20

Comparing DPIs — S30

Selecting projection data — S40

End

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5124914 A

**Non-patent literature cited in the description**

- **HENGYONG YU et al.** Data Consistency Based Rigid Motion Artifact Reduction in Fan-Beam CT. *IEEE TRANSACTIONS ON MEDICAL IMAGING,* 01 February 2007, vol. 26 (2), 249-260

- **KACHELRIESS MARC et al.** Kymogram detection and kymogram-correlated image reconstruction from subsecond spiral computed tomography scans of the heart. *MEDICAL PHYSICS,* 01 July 2002, vol. 29 (7), 1489-1503